# EUROPÄISCHE PATENTSCHRIFT

(11) **EP 2 316 358 B1**
(45) Veröffentlichungstag und Bekanntmachung des Hinweises auf die Patenterteilung: **18.01.2012**
(21) Anmeldenummer: 10014001.1
(22) Anmeldetag: 27.10.2010
(51) Int. Cl.: A61B 17/29

(54) **Medizinisches Instrument**
Medical instrument
Instrument médical

(30) Priorität: 31.10.2009 DE 102009051515
(43) Veröffentlichungstag der Anmeldung: 04.05.2011
(73) Patentinhaber: Richard Wolf GmbH, 75438 Knittlingen (DE)
(72) Erfinder: Boebel, Manfred, 75245 Bauschlott (DE); Prestel, Stephan, 76287 Rheinstetten-Mörsch (DE); Körner, Eberhard, Dipl.-Ing., 75438 Knittlingen (DE); Falk, Ernst, 75447 Sternenfels-Diefenbach (DE)
(74) Vertreter: Vollmann, Heiko

(56) Entgegenhaltungen:
- US-A1- 2003 236 549
- US-A1- 2009 088 792

## Beschreibung

Die Erfindung betrifft ein medizinisches Instrument mit den im Oberbegriff des Anspruchs 1 angegebenen Merkmalen.

In US 2003/0236549 A1 ist ein endoskopisches Instrument beschrieben, bei dem distalseitig einer Handhabe ein Schaft angeordnet ist. An dem distalen Ende des Schaftes ist ein Werkzeug schwenkbar befestigt. Das Werkzeug weist einen Hauptkörper auf, an dessen distalem Ende ein Zangenmaul ausgebildet ist. Der Hauptkörper des Werkzeugs ist zweiteilig, wobei ein distaler Abschnitt des Hauptkörpers relativ zu einem proximalen Abschnitt des Hauptkörpers um eine gemeinsame Mittelachse von proximalem und distalem Abschnitt drehbar ist. An der Handhabe sind Betätigungselemente in Form eines Drehrades und eines Kippschalters ausgebildet. In einer ersten Schaltstellung des Kippschalters ist das Drehrad derart mit dem Werkzeug bewegungsgekoppelt, dass das gesamte Werkzeug um eine Achse quer zur Mittelachse des Schaftes relativ zu dem Schaft verschwenkbar ist. In einer zweiten Schaltstellung des Kippschalters ist das Drehrad so mit dem Werkzeug bewegungsgekoppelt, dass der distale Abschnitt des Hauptkörpers des Werkzeugs relativ zu dem proximalen Abschnitt des Hauptkörpers verdrehbar ist.

Aus DE 10 2008 015 418 A1 ist ein endoskopisches Instrument bekannt, das distalseitig einer Handhabe einen Schaft mit einem abgewinkelten distalen Endabschnitt aufweist, wobei an dem distalen Schaftende wahlweise ein Zangen- oder Scherenmaulteil angeordnet ist. Bei diesem Instrument ist der Schaft relativ zu einer Längsachse des Schafts drehbar. Die Drehbarkeit des Schafts ermöglicht ein Wegschwenken des distalen Schaftabschnitts von dem Operationsgegenstand, um größere Freiräume in dem Operationsgebiet zu schaffen.

Zur Drehung des Schaftes ist dieser mit einem an der Handhabe angeordneten Drehrad bewegungsgekoppelt. Darüber hinaus ist auch das Zangen- oder Scherenmaul relativ zu dem distalen Endabschnitt des Schafts drehbar. Hierzu ist an der Handhabe ein zweites mit dem Maul bewegunsgekoppeltes Drehrad vorgesehen. Die beiden Drehräder weisen eine gemeinsame Drehachse auf und sind bezogen auf diese Drehachse an der Handhabe voneinander beabstandet angeordnet. Ein erstes proximalseitig angeordnetes Drehrad wird von einer das Instrument haltenden Person mit dem Daumen und ein davon distal beabstandetes Drehrad mit dem Zeigefinger bedient. Hierbei erweist es sich als problematisch, dass das mittels des Daumens bediente Drehrad bei der Bedienung des davon distal beabstandeten Drehrads von dem Zeigefinger umgriffen werden muss, wobei das proximalseitige Drehrad unerwünschterweise bewegt werden kann. Ein weiterer Nachteil dieses Instruments ist darin zu sehen, dass es die Verwendung von zwei an der Handhabe hintereinander angeordneten Drehrädern erschwert, die Handhabe an die unterschiedlichen Handgrößen einer möglichst groBen Anzahl von Benutzern des Instruments anzupassen.

Vor diesem Hintergrund liegt der Erfindung die Aufgabe zugrunde, ein medizinisches Instrument der vorbeschriebenen Art zu schaffen, das eine ergonomischere Handhabung auch bei unterschiedlicher Handgröße der Benutzer erlaubt.

Gelöst wird diese Aufgabe durch ein medizinisches Instrument mit den in Anspruch 1 angegebenen Merkmalen. Vorteilhafte Weiterbildungen dieses Instruments ergeben sich aus den Unteransprüchen, der nachfolgenden Beschreibung sowie der Zeichnung. Hierbei können gemäß der Erfindung die in den Unteransprüchen und der Beschreibung angegebenen Merkmale jeweils für sich aber auch in Kombination die erfindungsgemäße Lösung gemäß Anspruch 1 weiter ausgestalten.

Das erfindungsgemäße medizinische Instrument weist proximalseitig eine Handhabe und einen an der Handhabe drehbar angeordneten Schaft auf. Bei dem Schaft handelt es sich bevorzugt um einen solchen Schaft, bei dem ein distaler Endabschnitt schräg zu der Längsachse des Schafts in einem proximalen Endabschnitt gebogen ist. An dem distalen Ende des Schafts ist ein Werkzeug angeordnet. Bei dem Werkzeug kann es sich um ein Werkzeug mit einem Werkzeugmaul, wie eine Zange oder Schere, ein Koagulationswerkzeug oder ähnliches handeln. Das Werkzeug ist relativ zu dem Schaft drehbar.

Gemäß der Erfindung ist an der Handhabe ein Betätigungselement angeordnet, das in zumindest zwei Schaltstellungen bewegbar ist, wobei es in einer ersten Schaltstellung mit dem Schaft und in einer zweiten Schaltstellung mit dem Werkzeug bewegungsgekoppelt ist. Derart ausgebildet, ermöglicht es das erfindungsgemäße Instrument mit lediglich einem Betätigungselement sowohl den Schaft des Instruments in eine gewünschte Position relativ zu der Handhabe des Instruments zu drehen, als auch das an dem distalen Ende des Schafts angeordnete Werkzeug relativ zu dem Schaft und relativ zu der Handhabe zu drehen. Das Betätigungselement kann an der Handhabe in einem Bereich angeordnet sein, in dem es von den Anwendern unabhängig von deren Handgröße bequem bedient werden kann. Weiter vorteilhaft kann das Betätigungselement neben den Schaltstellungen, in denen es mit dem Schaft oder mit dem Werkzeug bewegungsgekoppelt ist, auch in mindestens eine weitere Schaltstellung bewegbar sein, in der das Betätigungselement weder mit dem Schaft, noch mit dem Werkzeug bewegungsgekoppelt ist oder in weitere Schaltstellungen bewegbar sein, um mit dem Betätigungselement in diesen zusätzlichen Schaltstellungen zusätzliche Funktionen auszuführen.

Das Betätigungselement ist zur Bewegungsübertragung auf den Schaft und auf das Werkzeug bevorzugt jeweils um eine Drehachse drehbar. Das heißt, je nachdem in welcher Schaltstellung sich das Betätigungselement befindet, ist durch eine Drehung des Betätigungselements entweder der Schaft oder das Werkzeug drehbar. Weiter bevorzugt ist das Betätigungselement in Richtung seiner Drehachse in seine Schaltstellungen axial verschiebbar. Dementsprechend kann das Betätigungselement normal zu der Ebene, in der es verdreht werden kann, in eine Position bewegt werden, in der eine Bewegungskopplung mit dem Schaft erfolgt und in eine weitere Position, in der eine Bewegungskopplung mit dem Werkzeug erfolgt. Als Betätigungselement kann vorteilhaft ein Drehrad vorgesehen sein, das vorzugsweise als ein Sternrad ausgebildet sein kann.

Zur Bewegungskopplung des Betätigungselements mit dem Schaft kann das Betätigungselement vorteilhaft ein erstes Kupplungsteil aufweisen, das mit einem mit dem Schaft bewegungsgekoppelten Kupplungsteil einen Formschluss bildet. Zweckmäßigerweise weist das erste Kupplungsteil des Betätigungselements solche Formschlussmittel auf, die durch ein Verschieben des Betätigungselements in Richtung dessen Drehachse mit korrespondierend ausgebildeten Formschlussmitteln des mit dem Schaft bewegungsgekoppelten Kupplungsteils in Eingriff bzw. außer Eingriff bringbar sind.

Um in der zweiten Schaltstellung des Betätigungselements auch eine Bewegungsübertragung von dem Betätigungselement auf das Werkzeug zu ermöglichen, kann das Betätigungselement weiter vorteilhaft ein zweites Kupplungsteil aufweisen, welches mit einem mit dem Werkzeug bewegungsgekoppelten Kupplungsteil einen Formschluss bildet. Auch in diesem Fall weist das zweite Kupplungsteil des Betätigungselements zweckmäßigerweise solche Formschlussmittel auf, die durch ein Verschieben des Betätigungselements in Richtung dessen Drehachse in korrespondierende Formschlussmittel des mit dem Werkzeug bewegungsgekoppelten Kupplungsteils eingreifen können.

In einer weiteren vorteilhaften Ausgestaltung des erfindungsgemäßen Instruments kann das Betätigungselement ein federvorgespanntes Rastelement aufweisen, das in der ersten Schaltstellung des Betätigungselements in eine erste an dem Schaft ausgebildete umfängliche Ringnut und in der zweiten Schaltstellung in eine zweite an dem Schaft ausgebildete umfängliche Ringnut eingreift. So kann in dem Betätigungselement bevorzugt ein Rastelement derart angeordnet sein, dass es von einem Federelement radial in Richtung des Schaftes gedrückt wird und bei einer Bewegung des Betätigungselements in Richtung dessen Drehachse bei Erreichen einer der beiden an dem Schaft an dessen Außenumfang ausgebildeten Ringnuten in diese Ringnut eingreift. Die Ringnuten sind bei dieser Ausgestaltung zweckmäßigerweise derart an dem Schaft angeordnet, dass das Betätigungselement beim Eingreifen des Rastelements in eine der Ringnuten entweder mit dem Schaft oder mit dem Werkzeug bewegungsgekoppelt ist. Beim Eingreifen des Rastelements in die Ringnuten ist das Betätigungselement entweder in der ersten oder in der zweiten Schaltstellung in axialer Richtung kraftund formschlüssig festgelegt, wobei eine Drehung des Betätigungselements und damit eine Bewegungsübertragung von dem Bewegungselement auf den Schaft bzw. auf das Werkzeug gewährleistet ist. Unbeabsichtigte Axialbewegungen des Betätigungselements aus den Schaltstellungen werden vorteilhaft verhindert. Wenn mit dem Betätigungselement zusätzliche Funktionen ausgeführt werden sollen oder wenn das Betätigungselement in einer Neutralstellung, in der es keine Funktion ausführt, festgelegt werden soll, können typischerweise an dem Schaft weitere Ringnuten vorgesehen sein, in die das Rastelement dann eingreifen kann.

Bevorzugt bildet das erste Kupplungsteil des Betätigungselements mit dem mit dem Schaft bewegungsgekoppelten Kupplungsteil eine erste Zahnkupplung und das zweite Kupplungsteil des Betätigungselements mit dem mit dem Schaft bewegungsgekoppelten Kupplungsteil eine zweite Zahnkupplung. Hierzu bildet zweckmäßigerweise ein mit einer Außenverzahnung versehener erster Zahnkranz das erste Kupplungsteil des Betätigungselements und ein mit einer Außenverzahnung versehener zweiter Zahnkranz das zweite Kupplungsteil des Betätigungselements.

Korrespondierend zu dem ersten Zahnkranz des Betätigungselements ist auf dem Schaft bevorzugt eine erste Nabe mit zwei voneinander radial beabstandeten Ringabschnitten angeordnet, wobei an der Innenseite eines äußeren der beiden Ringabschnitte eine mit der Verzahnung des ersten Zahnkranzes korrespondierende Verzahnung ausgebildet ist. Durch eine Axialbewegung des Betätigungselements in Richtung dessen Drehachse ist der erste Zahnkranz des Betätigungselements in den Zwischenraum zwischen dem inneren und dem äußeren Ringabschnitt der ersten Nabe einschiebbar, wobei die an dem ersten Zahnkranz des Betätigungselements ausgebildete Außenverzahnung mit der an dem äußeren Ringabschnitt der ersten Nabe ausgebildeten Innenverzahnung in Eingriff kommt, sodass in Drehrichtung des Betätigungselements ein Formschluss zwischen dem Betätigungselement und der ersten Nabe, das heißt dem mit dem Schaft bewegungsgekoppelten ersten Kupplungsteil entsteht.

Zweckmäßigerweise ist auch eine mit dem Werkzeug bewegungsgekoppelte Nabe vorgesehen, die mit dem zweiten Zahnkranz des Betätigungselements eine Zahnkupplung bildet. Diese zweite Nabe ist bevorzugt an einem Rohr angeordnet, an dessen distalen Ende das Werkzeug angeordnet ist. Das Rohr ist zweckmäßigerweise in dem Schaft drehbeweglich angeordnet. Vorteilhaft weist die zweite Nabe ebenfalls zwei voneinander radial beabstandete Ringabschnitte auf, wobei an der Innenseite eines äußeren Ringabschnitts eine mit der Verzahnung des zweiten Zahnkranzes korrespondierende Verzahnung ausgebildet ist.

Besonders vorteilhaft verjüngen sich die Zähne des ersten Zahnkranzes in axiale Richtung zu dem mit dem Schaft bewegungsgekoppelten Kupplungsteil und die Zähne des zweiten Zahnkranzes in axialer Richtung zu dem mit dem Werkzeug bewegungsgekoppelten Kupplungsteil. Dementsprechend weisen die radial nach außen ragenden Zähne der beiden Zahnkränze in Richtung der Drehachse des Betätigungselements eine keilförmige Form auf, die den Eingriff in die Verzahnung des mit dem Schaft bewegungsgekoppelten Kupplungsteils bzw. mit dem mit dem Werkzeug bewegungsgekoppelten Kupplungsteils erleichtern.

Zur Bewegungskopplung der ersten Nabe mit dem Schaft weist der Schaft vorteilhafterweise in dem Bereich, in dem die erste Nabe angeordnet ist, eine Außenquerschnittskontur mit zumindest einem gerade abgeflachten Abschnitt auf, wobei die von dem inneren Ringabschnitt begrenzte Innenquerschnittskontur der ersten Nabe mit der Außenquerschnittskontur des Schaftes korrespondiert. Auf diese Weise wird in der Drehebene von Schaft und erster Nabe zwischen dem Schaft und der ersten Nabe ein Formschluss geschaffen. Bevorzugt weist der Schaft in dem Bereich, in dem die erste Nabe angeordnet ist, einen oktogonalen Außenquerschnitt und die erste Nabe korrespondierend ebenfalls einen oktogonalen Innenquerschnitt auf.

Um die zweite Nabe an dem in dem Schaft geführten Rohr in der Drehrichtung von Rohr und zweiter Nabe formschlüssig festzulegen, weist auch das in dem Schaft geführte Rohr in dem Bereich, in dem die zweite Nabe an dem Rohr angeordnet ist, bevorzugt eine Außenquerschnittskontur mit zumindest einem gerade abgeflachten Abschnitt auf, wobei die von dem inneren Ringabschnitt begrenzte Innenquerschnittskontur der zweiten Nabe mit der Außenquerschnittskontur des Rohres korrespondiert. Auch hier ist bevorzugt vorgesehen, dass das Rohr in dem Bereich, in dem die zweite Nabe angeordnet ist, einen oktogonalen Außenquerschnitt und die zweite Nabe einen oktogonalen Innenquerschnitt aufweist.

Vorteilhaft können bei dem erfindungsgemäßen Instrument das mit dem Schaft bewegungsgekoppelte erste Kupplungsteil und das mit dem Werkzeug gekoppelte Kupplungsteil dann, wenn das jeweilige Kupplungsteil nicht mit dem Betätigungselement bewegungsgekoppelt ist, an der Handhabe gegen eine Drehbewegung festlegbar sein. Hierzu können beide Kupplungsteile beispielsweise in Verschieberichtung des Betätigungselements verschiebbar sein, wobei die Kupplungsteile Formschlussmittel aufweisen, mit denen sie in einer Verschiebestellung mit der Handhabe drehfest verbindbar sind. In diese Verschiebestellung werden die Kupplungsteile vorteilhaft von einem jeweils an den Kupplungsteilen angeordneten Federelement gedrückt. Zum Verdrehen des ersten Kupplungsteils mit dem damit bewegungsgekoppelten Schaft bzw. zum Verdrehen des mit dem Werkzeug bewegungsgekoppelten Kupplungsteils, kann das jeweilige Kupplungsteil von dem Betötigungselement gegen die Federkraft des Federelements in eine Stellung verschoben werden, in der es nicht mehr drehfest an der Handhabe festgelegt ist, das heißt, der Formschluss des jeweiligen Kupplungsteils mit der Handhabe wird hierdurch aufgehoben.

Darüber hinaus ist es zum verdrehsicheren Festlegen der beiden Kupplungsteile auch möglich, diese nicht verschiebbar in der Handhabe anzuordnen und an einer Außenseite der Kupplungsteile eine Vielzahl von Ausnehmungen vorzusehen, in die jeweils ein handhabeseitiges Rastelement kraft- und formschlüssig eingreifen kann und so die Kupplungsteile gegen ein unbeabsichtigtes Verdrehen sichert. Zum Verdrehen des Kupplungsteils ist dann die von dem Rastelement auf das Kupplungsteil ausgeübte Kraft zu überwinden.

Nachfolgend ist das erfindungsgemäße Instrument anhand in der Zeichnung dargestellter Ausführungsbeispiele näher erläutert. In der Zeichnung zeigt:
- Fig. 1: schematisch ein medizinisches Instrument in einer Seiten- ansicht,
- Fig. 2: vergrößert eine Handhabe des Instruments nach fig. 1 in einer teilgeschnittenen Seitenansicht,
- Fig. 3: eine erste Betätigungseinrichtung zum Drehen eines Schafts und eines Werkzeugs des Instruments nach Fig. 1 in perspektivischer Darstellung,
- Fig. 4: die Betätigungseinrichtung nach Fig. 3 in einer Unteran- sicht,
- Fig. 5: die Betätigungseinrichtung nach Fig. 3 in einer Frontan- sicht,
- Fig. 6: die Betätigungseinrichtung nach Fig. 3 in einer Schnittan- sicht entlang der Linie VI - VI in Fig. 5
- Fig. 7: ein Betätigungselement der Betätigungseinrichtung nach Fig. 3 in perspektivischer Darstellung,
- Fig. 8: das Betätigungselement nach Fig. 7 in einer Frontansicht,
- Fig. 9: das Betätigungselement nach Fig. 7 in einer Seitenansicht,
- Fig. 10: eine Schnittansicht entlang der Linie X - X in Fig. 9,
- Fig. 11: eine Nabe der Betätigungseinrichtung nach Fig. 3 in per- spektivischer Darstellung,
- Fig. 12: die Nabe nach Fig. 11 in einer Frontansicht,
- Fig. 13: die Nabe nach Fig. 11 in einer Seitenansicht,
- Fig. 14: eine Schnittansicht entlang der Linie XIV - XIV in Fig. 13,
- Fig. 15: die Nabe nach Fig. 11 in einer Rückansicht,
- Fig. 16: eine zweite Nabe der Betätigungseinrichtung nach Fig. 3 in perspektivischer Darstellung,
- Fig. 17: die Nabe nach Fig. 16 in einer Frontansicht,
- Fig. 18: die Nabe nach Fig. 16 in einer Seitenansicht,
- Fig. 19: eine Schnittansicht entlang der Linie XIX - XIX in Fig. 18,
- Fig. 20: die Nabe nach Fig. 16 in einer Rückansicht,
- Fig. 21: eine zweite Betätigungseinrichtung zum Drehen eines Schaftes und eines Werkzeugs des Instruments nach Fig. 1 in perspektivischer Darstellung,
- Fig. 22: die Betätigungseinrichtung nach Fig. 21 in einer Seitenan- sicht,
- Fig. 23: die Betätigungseinrichtung nach Fig. 21 in einer Frontan- sicht,
- Fig. 24: die Betätigungseinrichtung nach Fig. 21 in einer Unteran- sicht,
- Fig. 25: eine Schnittansicht entlang der Linie XXV - XXV in Fig. 24,
- Fig. 26: eine Schnittansicht entlang der Linie XXVI - XXVI in Fig. 23,
- Fig. 27: eine Nabe der Betätigungseinrichtung nach Fig. 21 in per- spektivischer Darstellung,
- Fig. 28: die Nabe nach Fig. 27 in einer Frontansicht,
- Fig. 29: die Nabe nach Fig. 27 in einer Seitenansicht,
- Fig. 30: eine Schnittansicht entlang der Linie XXX - XXX in Fig. 29, und
- Fig. 31: die Nabe nach Fig. 27 in einer Rückansicht.

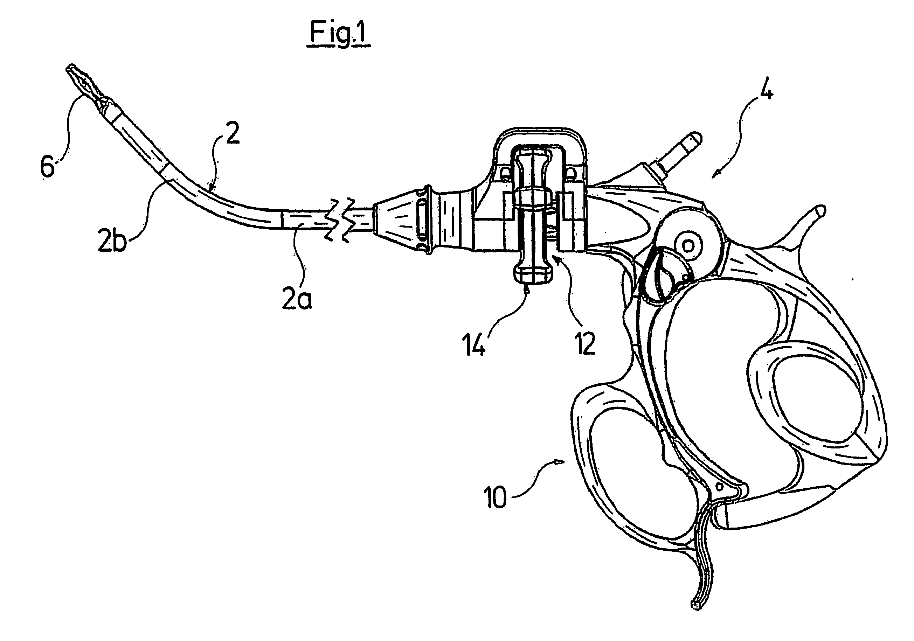

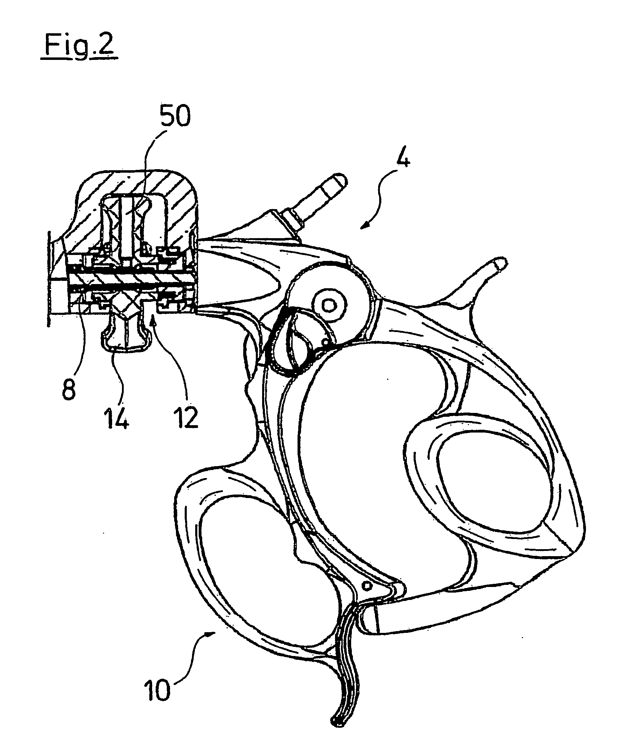

Bei dem in Fig. 1 dargestellten medizinischen Instrument handelt es sich um ein Hohlschaftinstrument mit einem starren Schaft 2, an dem in üblicher Weise proximalseitig eine Handhabe 4 angeordnet ist. Der Schaft 2 weist einen gerade ausgerichteten proximalen Schaftabschnitt 2a und einen sich daran anschließenden schräg zum Schaftabschnitt 2a gebogenen distalen Schaftabschnitt 2b auf. An dem distalen Ende des Schafts 2 ist ein Werkzeug 6 in Form einer Fasszange angeordnet. Der Schaft 2 ist in der Handhabe 4 um die Längsachse des proximalen Schaftabschnitts 2a drehbar gelagert, also relativ zu der Handhabe 4 drehbar. Das Werkzeug 6 wiederum ist relativ zu dem Schaft 2 drehbar und ist hierzu an dem distalen Ende eines durch den Schaft 2 geführten Rohres 8 (Fig. 2) befestigt. Das Rohr 8 ist in dem Schaft 2 und der Handhabe 4 drehbar gelagert.

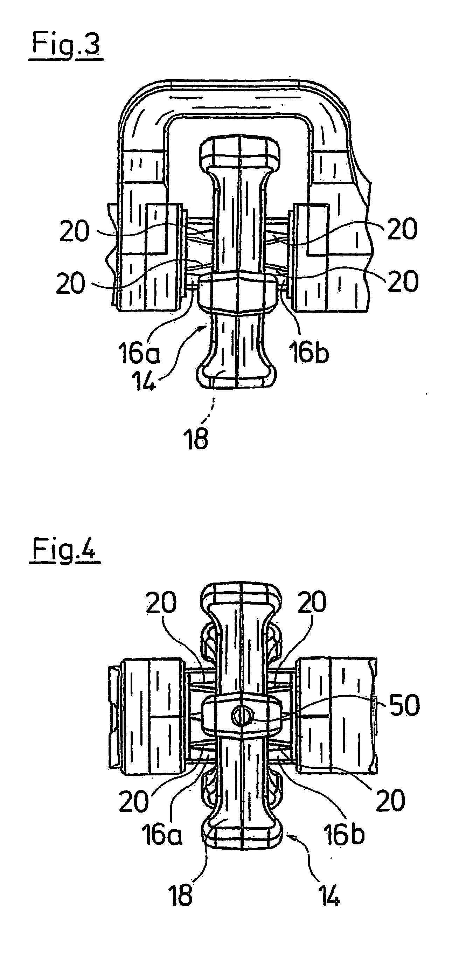

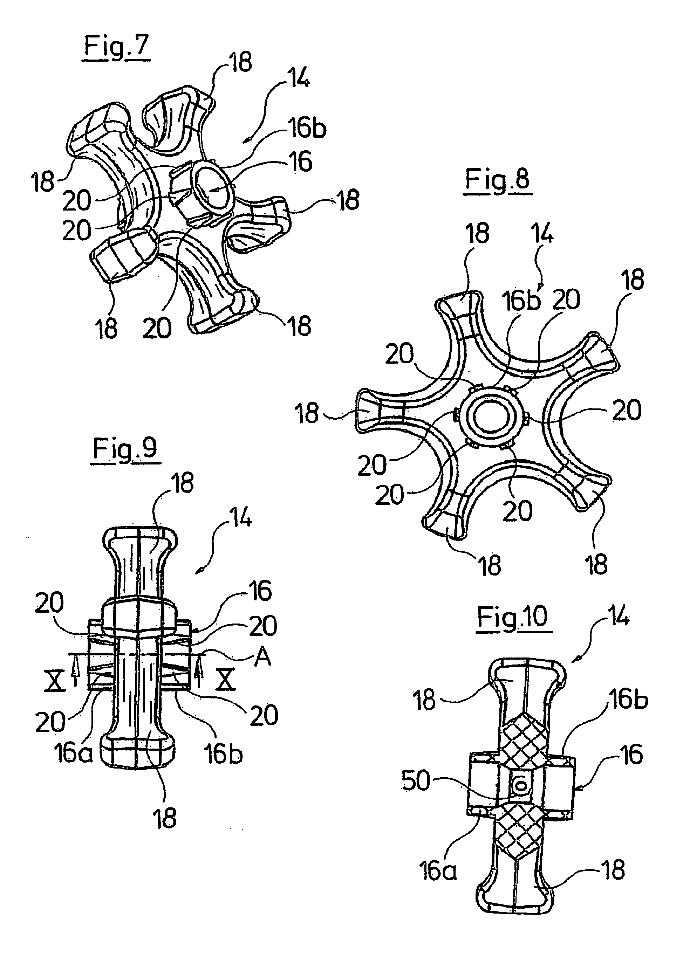

Zum Drehen des Schafts 2 und des Werkzeugs 6 weist die Handhabe 4 zwischen einem Griffteil 10 und dem distalen Ende der Handhabe 4, an der der Schaft 2 aus der Handhabe 4 herausgeführt ist, eine Betätigungseinrichtung 12 auf. Eine solche Betätigungseinrichtung 12 ist in den Figuren 3 bis 6 in verschiedenen Ansichten dargestellt. Zum manuellen Bedienen der Betätigungseinrichtung 12 weist diese ein Bedienelement 14 auf. Wie insbesondere den Fig. 7 bis 10, in denen das Bedienelement 14 separat dargestellt ist, zu entnehmen ist, bildet ein Sternrad 14 das Betätigungselement 14.

Das Sternrad 14 weist einen hülsenförmigen Grundkörper 16 auf, an dessen äußerer Mantelfläche über deren Umfang gleichmäßig verteilt fünf Betötigungsarme 18 radial nach außen kragen. In Richtung einer Längsachse A des Sternrads 14 ragen Abschnitte 16a und 16b des Grundkörpers 16 über die Außenseiten der Betätigungsarme 18 hinaus. An den Umfangsflächen dieser Abschnitte 16a und 16b des Grundkörpers 16 sind, über den Umfang der Abschnitte 16a und 16b gleichmäßig verteilt, jeweils sechs Zähne 20 ausgebildet, die radial nach außen kragen. Insofern bilden die Abschnitte 16a und 16b des Grundkörpers 16 Zahnkränze 16a und 16b. In Richtung der Längsachse A des Sternrads 14 weisen die Zähne 20 eine Keilform auf, wobei sie sich jeweils zu den Stirnseiten der Abschnitte 16a und 16b des Grundkörpers 16 hin verjüngen.

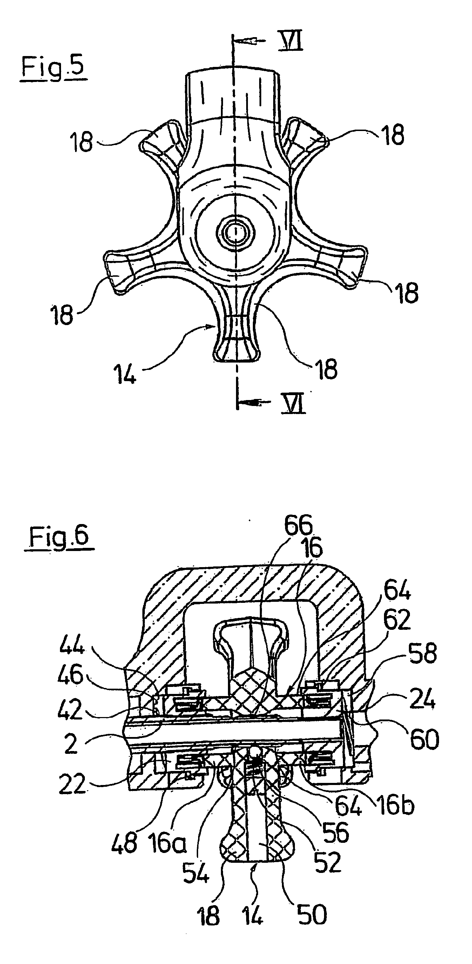

In der Betätigungseinrichtung 12 ist das Sternrad 14 auf dem dort drehbar geführten Schaft 2 des Instruments drehbar gelagert und in einem begrenzten Bereich in Richtung der Längsachse A des Sternrades 14 auf dem Schaft 2 verschiebbar (Fig. 6). In distaler Richtung wird der axiale Verschiebeweg des Sternrads 14 durch eine Nabe 22 begrenzt, die auf dem Schaft 2 angeordnet ist, während eine Nabe 24, die auf dem Rohr 8 in einem Bereich angeordnet ist, der aus dem Schaft 2 herausragt, den Verschiebeweg des Sternrads 18 in proximaler Richtung begrenzt.

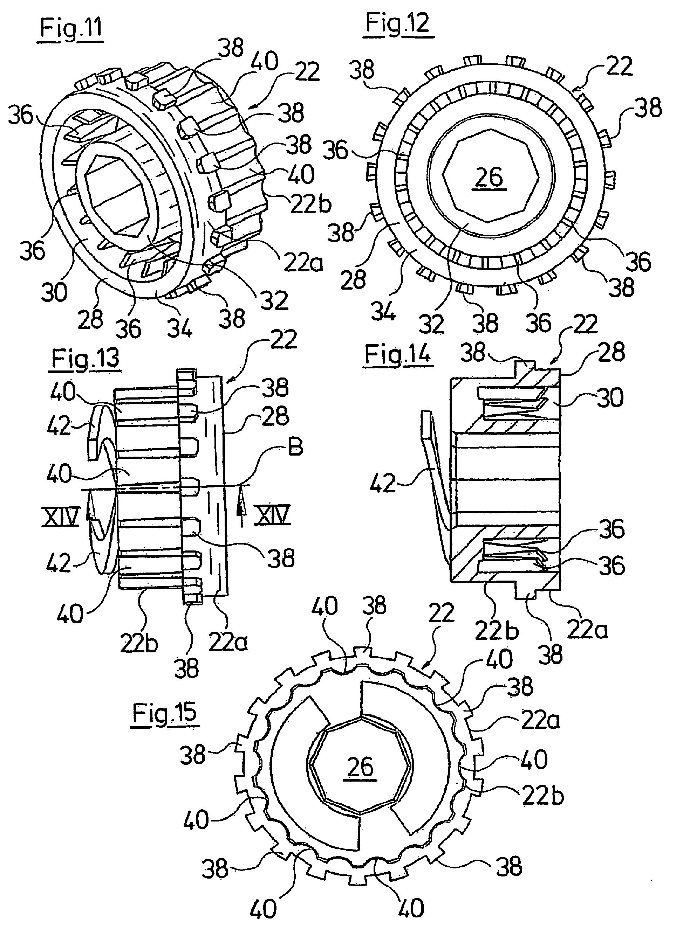

Die in den Fig. 11 bis 15 separat dargestellte Nabe 22 weist einen oktogonalen Innenquerschnitt 26 auf. Korrespondierend weist der Schaft 2 in einem Bereich, in dem die Nabe 22 angeordnet werden soll, einen dementsprechenden oktogonalen Außenquerschnitt auf. Aufgrund dieser Ausgestaltung des Innenquerschnitts 26 der Nabe 22 und des Außenquerschnitts des Schaftes 2 wird in Drehrichtung des Schaftes 2 ein Formschluss zwischen der Nabe 22 und dem Schaft 2 geschaffen, sodass sich die Nabe 22 nicht relativ zu dem Schaft 2 verdrehen kann und nur eine gleichzeitige Verdrehung von Schaft 2 und Nabe 22 möglich ist.

An einer Stirnseite 28 der Nabe 22 ist eine Ringnut 30 ausgebildet, die konzentrisch zu dem Innenquerschnitt 26 der Nabe 22 angeordnet ist. Die Ringnut 30 bildet einen inneren Ringabschnitt 32 und einen davon radial beabstandeten äußeren Ringabschnitt 34. An der Innenseite des äußeren Ringabschnitts 34 ist eine Vielzahl nach innen gerichteter Zähne 36 gleichmäßig über den Innenumfang des äußeren Ringabschnitts 34 verteilt angeordnet. In Richtung einer Längsachse B der Nabe 22 und in Richtung der Stirnseite 28 verjüngen sich die Zähne 36 keilförmig.

Der Außenquerschnitt der Nabe 22 ist nicht konstant. Die Nabe 22 ist abgestuft ausgebildet, mit einem ersten sich an die Stirnseite 28 anschließenden Nabenabschnitt 22a, dessen Außendurchmesser größer als der eines sich an den Nabenabschnitt 22a anschließenden Nabenabschnitts 22b ist. Dort, wo der Nabenabschnitt 22a in den Nabenabschnitt 22b übergeht, ist an dem Außenumfang des Nabenabschnitts 22a eine Vielzahl von Zähnen 38 gleichmäßig über den Umfang verteilt angeordnet. Die Zähne 38 kragen an dem Nabenabschnitt 22a radial nach außen. An dem Nabenabschnitt 22b ist eine Vielzahl von Nuten 40 ausgebildet. Diese Nuten 40 erstrecken sich über den gesamten Umfang des Nabenabschnitts 22b parallel zu der Längsachse B der Nabe 22. Die Nuten 40 weisen eine konkav nach innen gewölbte Form auf. An der von der Stirnseite 28 abgewandten Stirnseite der Nabe 22 sind zwei Blattfedern 42 derart angeordnet, dass deren Federkraft parallel zur Längsachse B der Nabe 2 gerichtet ist.

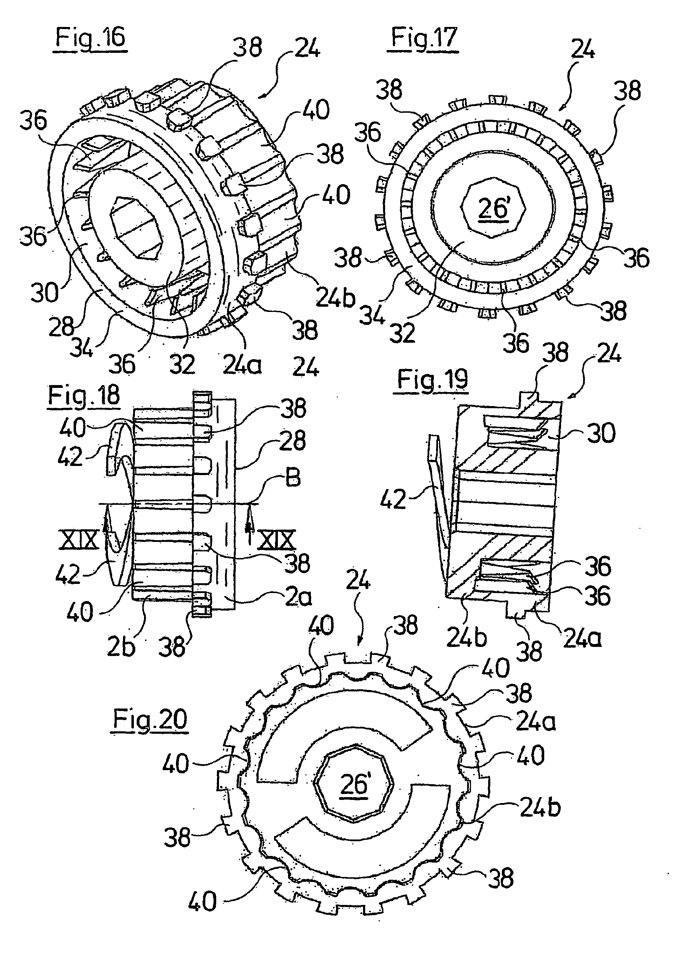

Die in den Figuren 16 bis 20 separat dargestellte Nabe 24 unterscheidet sich von der Nabe 22 dahingehend, dass der oktogonale Innenquerschnitt 26' der Nabe 24 kleiner als der Innenquerschnitt 26 der Nabe 22 ist. Ansonsten stimmen die Naben 22 und 24 hinsichtlich aller anderen Merkmale überein.

Die Anordnung der Nabe 22 auf dem Schaft 2 geht aus Fig. 6 hervor. Wie bereits angemerkt worden ist, ist die Nabe 22 auf dem Schaft 2 in einem Bereich angeordnet, in dem der Schaft 2 korrespondierend zu dem Innenquerschnitt 26 der Nabe 22 einen oktogonalen Außenquerschnitt aufweist. Dieser oktogonale Bereich des Schaftes 2 ist größer als die Abmessung der Nabe 22 in Richtung ihrer Längsachse B, sodass die Nabe 22 in diesem Bereich auf dem Schaft 2 axial verschiebbar ist. Die Betätigungseinrichtung 12 weist eine Ausnehmung 44 auf, in die die Nabe 22 eingreift. Hierbei stützen sich die Blattfedern 42 der Nabe 22 an einer die Ausnehmung 44 begrenzenden Stirnflächen 46 ab. Die von den Zähnen 38 am Außenumfang des Nabenabschnitts 22a gebildete Außenverzahnung ist hierbei mit einer an der Innenseite der Ausnehmung 44 korrespondierend ausgebildeten Innenverzahnung im Eingriff. Hierdurch ist die Nabe 22 gegen eine Drehung um ihre Längsachse B formschlüssig festgelegt. Das heißt, in dieser Stellung der Nabe 22 kann der Schaft 2 nicht gedreht werden.

Zum Drehen des Schaftes 2 wird das Sternrad 14, das proximalseitig der Nabe 22 angeordnet ist, in Richtung der Nabe 22 verschoben, wobei der Zahnkranz 16a des Sternrads 14 in die an der Nabe 22 ausgebildete Ringnut 30 eingreift und die Nabe 22 gegen die Federkraft der Blattfedern 42 weiter in die Ausnehmung 44 hinein schiebt. Hierdurch wird die von den Zähnen 38 gebildete Außenverzahnung des Nabenabschnitts 22a in eine an der Ausnehmung in Verschieberichtung hinter der Innenverzahnung ausgebildete Ringnut 48 verschoben, sodass die Nabe 22 in dieser Position drehbar ist. Um die Nabe 22 mit dem darin in Eingriff befindlichen Sternrad 14 in dieser Position festlegen zu können, ist in einem der Betätigungsarme 18 des Sternrads 14 eine in radialer Richtung verlaufende Bohrung 50 ausgebildet, in der ein Rastelement 52 gelagert ist. An dem Schaft 2 ist eine konkav nach innen gewölbte Ringnut 54 ausgebildet. In diese Ringnut 54 greift eine Kugel 56 des Rastelements 52 ein, wenn die Nabe 22 von dem Sternrad 14 soweit verschoben worden ist, dass sich die an dem Nabenabschnitt 22a angeordneten Zähne 38 in der an der Ausnehmung 44 ausgebildeten Ringnut 48 befinden. Die von dem Rastelement 52 ausgeübte Federkraft ist so groß, dass die von den Blattfedern 42 ausgeübte Federkraft nicht ausreicht, die Nabe 22 aus dieser Position heraus zu bewegen. Da die Nabe 22 und das Sternrad 14 eine Zahnkupplung bilden, kann der Schaft 2 in dieser Stellung durch Drehung des Sternrades 14 gedreht werden.

Proximalseitig des Sternrads 14 ist in der Betätigungseinrichtung 12 die Nabe 24 auf einem aus dem Schaft 2 herausragenden Abschnittes 8 angeordnet. In diesem Abschnitt weist das Rohr 8 einen mit dem Innenquerschnitt 26' der Nabe 24 korrespondierenden oktogonalen Au-Benquerschnitt auf. Die Nabe 24 ist in diesem Abschnitt des Rohres 8 axial verschiebbar. In der Betätigungseinrichtung 12 ist eine weitere Ausnehmung 58 ausgebildet, in die die Nabe 24 eingreift, wobei sich die Blattfedern 42 der Nabe 24 an einer die Ausnehmung 58 begrenzenden Stirnfläche 60 abstützen. Die von den Zähnen 38 am Außenumfang des Nabenabschnitts 24a gebildete Außenverzahnung ist hierbei mit einer an der Innenseite der Ausnehmung 58 korrespondierenden Innenverzahnung im Eingriff. Hierdurch ist die Nabe 24 gegen ein Drehen um ihre Längsachse B formschlüssig festgelegt, das heißt, in dieser Stellung der Nabe 24 kann das Rohr 8 nicht gedreht werden.

Zum Drehen des Rohres 8 wird das Sternrad 14, das distalseitig der Nabe 24 angeordnet ist, in Richtung der Nabe 24 verschoben, wobei der Zahnkranz 16b des Sternrades 14 in die an der Nabe 24 ausgebildete Ringnut 30 eingreift und die Nabe 24 gegen die Federkraft der Blattfedern 42 weiter in die Ausnehmung 58 hinein schiebt. Hierdurch wird die von den Zähnen 38 gebildete Außenverzahnung des Nabenabschnitts 24a in eine an der Ausnehmung in Verschieberichtung des Sternrads 14 vor der Innenverzahnung ausgebildete Ringnut 62 verschoben, sodass die Nabe 24 in dieser Position drehbar ist.

Korrespondierend zu dieser Position ist an dem Schaft 2 eine weitere konkav nach innen gewölbte Ringnut 64 ausgebildet. In diese Ringnut 64 greift die Kugel 56 des in dem Sternrad 14 angeordneten Rastelements 52 ein, wenn die Nabe 24 von dem Sternrad 14 so weit verschoben worden ist, dass sich die an dem Nabenabschnitt 24a angeordneten Zähne 38 in der an der Ausnehmung 44 ausgebildeten Ringnut 62 befinden. Die von dem Rastelement 52 ausgeübte Federkraft ist so groß, dass sie die von den Blattfedern 42 ausgeübte Federkraft übersteigt, sodass die von den Blattfedern 42 ausgeübte Federkraft nicht ausreicht, die Nabe 24 aus dieser Position heraus zu bewegen. Da die Nabe 24 und das Sternrad 14 eine Zahnkupplung bilden, kann das Rohr 8 in dieser Stellung durch Drehung des Sternrades 14 gedreht werden.

Zwischen den an dem Schaft 2 ausgebildeten Ringnuten 54 und 64 ist an dem Schaft 2 eine weitere Ringnut 66 ausgebildet. Wenn die Kugel 56 des Rastelements 52 in diese Ringnut 66 eingreift, wird das Sternrad 14 in einer Neutralstellung gehalten, in der es weder mit der Nabe 22 noch mit der Nabe 24 in Eingriff ist, sodass weder der Schaft 2 noch das an dem distalen Ende des Rohrs 8 angeordnete Werkzeug 6 durch eine Drehung des Sternrades 14 bewegt werden können.

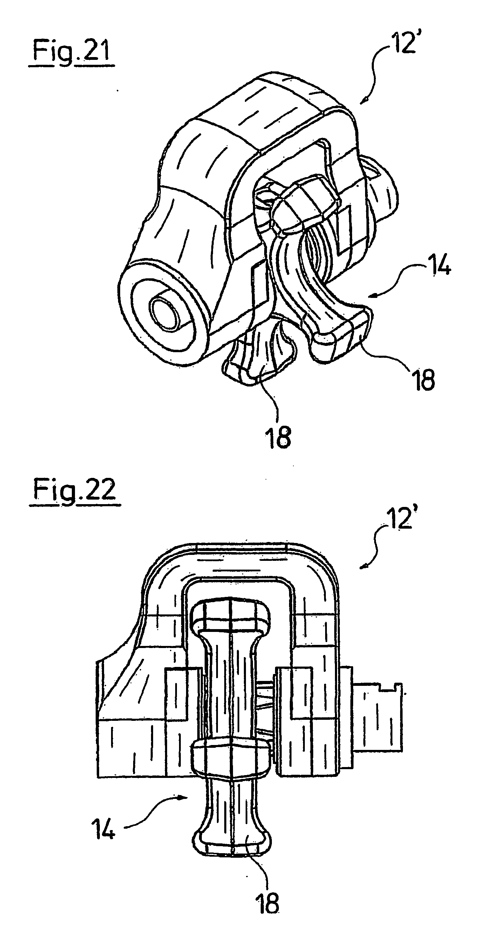

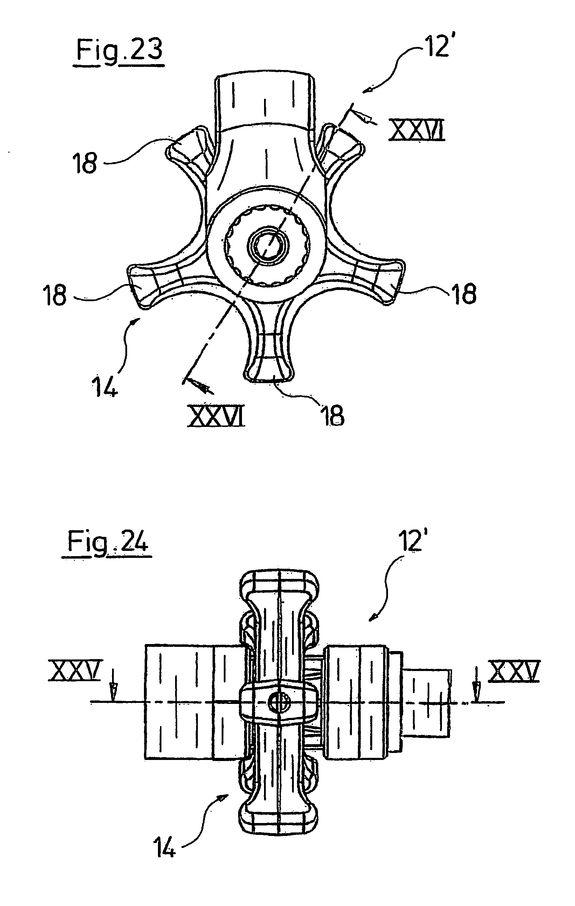

In den Figuren 21 bis 26 ist eine alternative Betätigungseinrichtung 12' zum Drehen des Schafts 2 und des Werkzeugs 6 dargestellt. Diese Betätigungseinrichtung 12' unterscheidet sich von der oben beschriebenen Betätigungseinrichtung 12 im Wesentlichen durch die Ausgestaltung der auf dem Schaft 2 und dem Rohr 8 angeordneten Naben, die zusammen mit dem Sternrad 14 Zahnkupplungen zum Drehen des Schafts 2 bzw. des Rohres 8 bilden und durch die Anordnungen dieser Naben in der Betätigungseinrichtung 12'.

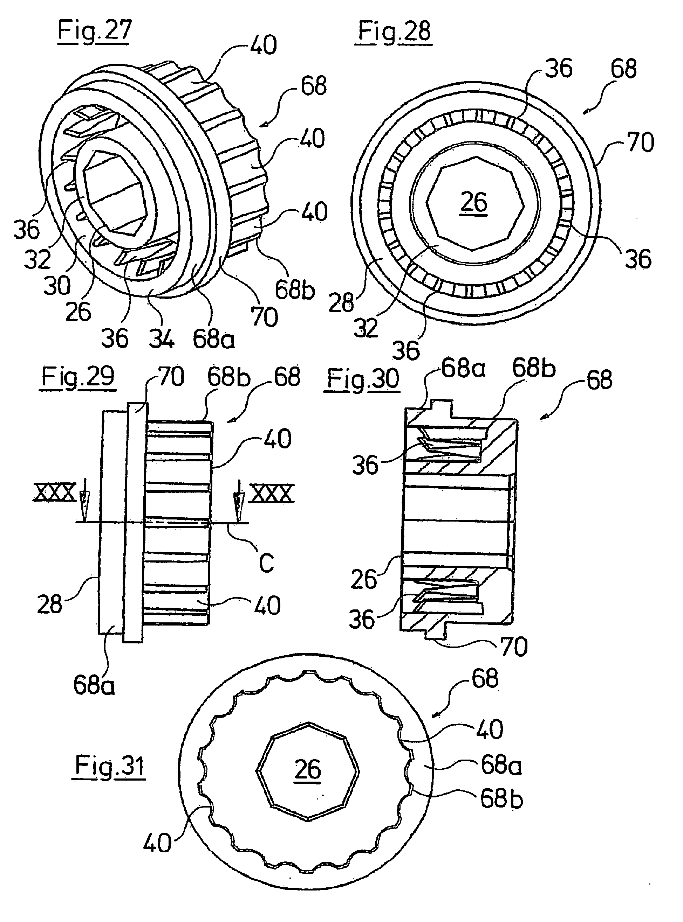

In der Betätigungseinrichtung 12' ist auf dem Schaft 2 eine Nabe 68 angeordnet, die in den Fig. 21 bis 27 in verschiedenen Ansichten separat dargestellt ist. Die Nabe 68 unterscheidet sich von der in den Fig. 11 bis 15 dargestellten Nabe 22 dadurch, dass bei der Nabe 68 die bei der Nabe 22 von den Zähnen 38 gebildete Außenverzahnung und die bei der Nabe 22 an der Stirnseite des Nabenabschnitts 22b angeordneten Federelemente 42 fehlen. Des Weiteren ragt an einem mit dem Nabenabschnitt 22a der Nabe 22 korrespondierenden Nabenabschnitt 68a der Nabe 68 statt der an dem Nabenabschnitt 22a der Nabe 22 ausgebildeten Verzahnung ein ringförmiger Vorsprung 70 radial nach außen.

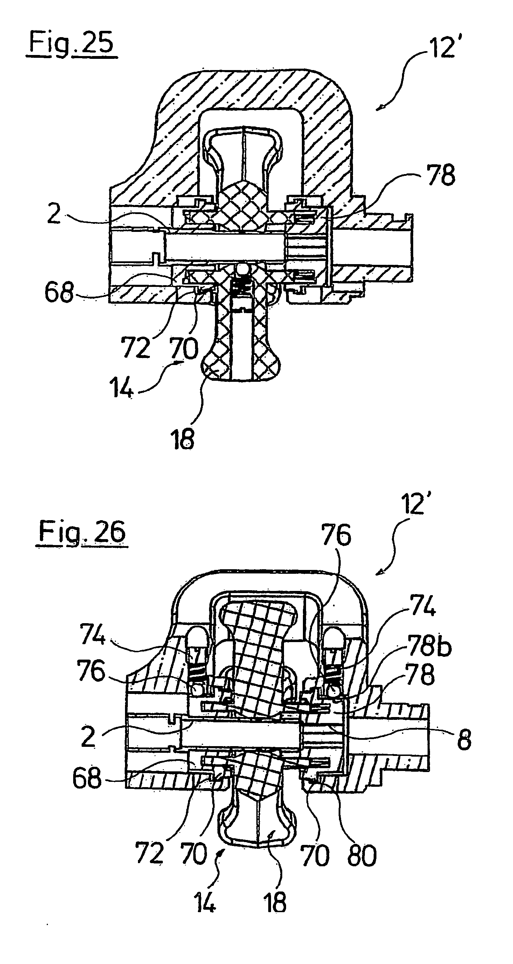

Die Anordnung der Nabe 68 auf dem Schaft 2 in der Betätigungseinrichtung 12' geht aus den Fig. 25 und 26 hervor. In der Betätigungseinrichtung 12' ist die Nabe 68 nicht verschiebbar angeordnet. Stattdessen greift der Vorsprung 70 der Nabe 68 in eine an der Betätigungseinrichtung 12' ausgebildete Ringnut 72 ein und wird auf diese Weise formschlüssig gegen eine Axialbewegung gesichert. Um die Nabe 68 bzw. den Schaft 2 gegen eine unbeabsichtigte Drehbewegung zu sichern, ist in der Betätigungseinrichtung 12' außenseitig eines Nabenabschnitts 68b, der mit dem Nabenabschnitt 22b der Nabe 22 korrespondiert, ein Rastelement 74 derart angeordnet, dass eine Rastkugel 76 des Rastelements 74 in die an dem Nabenabschnitt 68b ausgebildeten Nuten 40 eingreift. Wie bei der in den Fig. 3 bis 6 dargestellten Betätigungseinrichtung 12 ermöglicht der Eingriff des Zahnkranzes 16a des Sternrads in die an der Nabe 68 ausgebildete Verzahnung eine Bewegungskopplung des Sternrades 14 mit der Nabe 68 bzw. mit dem Schaft 2, wobei hierbei die von dem Rastelement 74 ausgeübte Federkraft überwunden werden muss.

Die Ausgestaltung des in der Betätigungseinrichtung 12' auf dem Rohr 8 angeordneten Nabe 78 entspricht der Ausgestaltung der Nabe 68 mit dem Unterschied, dass der Innenquerschnitt der Nabe 78 an den oktogonalen Außenquerschnitt des Rohres 8 angepasst ist. Auch die Nabe 78 ist in der Betätigungseinrichtung 12' nicht verschiebbar angeordnet. Stattdessen greift ein Vorsprung 70 der Nabe 78 in eine an der Betätigungseinrichtung 12' ausgebildete weitere Ringnut 80 ein und wird auf diese Weise formschlüssig gegen eine Axialbewegung gesichert.

Um die Nabe 78 bzw. das Rohr 8 gegen eine unbeabsichtigte Drehbewegung zu sichern, ist in der Betätigungseinrichtung 12' außenseitig eines Nabenabschnitts 78b, der mit dem Nabenabschnitt 68b der Nabe 68 korrespondiert, ein weiteres Rastelement 74 so angeordnet, dass die Rastkugel 76 dieses Rastelements 74 in die an dem Nabenabschnitt 68b ausgebildeten Nuten 40 eingreift. Wie bei der in den Fig. 3 bis 6 dargestellten Betätigungseinrichtung 12 ermöglicht der Eingriff des Sternrads 14 in die an der Nabe 78 ausgebildete Verzahnung eine Bewegungskopplung des Sternrades 14 mit der Nabe 78 bzw. mit dem Rohr 8. Zum Drehen des Rohrs 8 muss die von dem Rastelement 74 ausgeübte Federkraft überwunden werden, wobei die Rastkugel 76 des Rastelements 74 jeweils in die in Drehrichtung nächste Nut 40 eingreift und so eine abgestufte Drehbewegung ermöglicht.

### Bezugszeichenliste

- 2: - Schaft
- 2a, 2b: - Schaftabschnitt
- 4: - Handhabe
- 6: - Werkzeug
- 8: - Rohr
- 10: - Griffteil
- 12, 12': - Betätigungseinrichtung
- 14: - Betätigungselement, Sternrad
- 16: - Grundkörper
- 16a, 16b: - Abschnitt, Zahnkranz
- 18: - Betätigungsarm
- 20: - Zahn
- 22: - Nabe
- 22a, 22b: - Nabenabschnitt
- 24: - Nabe
- 24a, 24b: - Nabenabschnitt
- 26: - Innenquerschnitt
- 28: - Stirnseite
- 30: - Ringnut
- 32: - Ringabschnitt
- 34: - Ringabschnitt
- 36: - Zahn
- 38: - Zahn
- 40: - Nut
- 42: - Blattfeder
- 44: - Ausnehmung
- 46: - Stirnfläche
- 48: - Ringnut
- 50: - Bohrung
- 52: - Rastelement
- 54: - Ringnut
- 56: - Kugel
- 58: - Ausnehmung
- 60: - Stirnfläche
- 62: - Ringnut
- 64: - Ringnut
- 66: - Ringnut
- 68: - Nabe
- 68a, 68b: - Nabenabschnitt
- 70: - Vorsprung
- 72: - Ringnut
- 74: - Rastelement
- 76: - Rastkugel
- 78: - Nabe
- 78a, 78b: - Nabenabschnitt
- 80: - Ringnut
- A: - Längsachse
- B: - Längsachse

## Patentansprüche

1. Medizinisches Instrument mit einer proximalseitig angeordneten Handhabe (4), mit einem an der Handhabe (4) drehbar angeordneten Schaft (2) und mit einem am distalen Ende des Schafts (2) angeordneten Werkzeug (6), welches relativ zu dem Schaft (2) drehbar ist, **dadurch gekennzeichnet, dass** an der Handhabe (4) ein Betätigungselement (14) angeordnet ist, das in zumindest zwei Schaltstellungen bewegbar ist, wobei es in einer ersten Schaltstellung mit dem Schaft (2) und in einer zweiten Schaltstellung mit dem Werkzeug (6) bewegungsgekoppelt ist.

2. Medizinisches Instrument nach Anspruch 1, **dadurch gekennzeichnet, dass** das Betätigungselement (14) zur Bewegungsübertragung auf den Schaft (2) und auf das Werkzeug (6) jeweils um eine gemeinsame Drehachse drehbar ist und in Richtung der Drehachse in seine Schaltstellungen axial verschiebbar ist.

3. Medizinisches Instrument nach einem der vorangehenden Ansprüche, **dadurch gekennzeichnet, dass** das Betätigungselement (14) ein erstes Kupplungsteil aufweist, welches mit einem mit dem Schaft (2) bewegungsgekoppelten Kupplungsteil einen Formschluss bildet.

4. Medizinisches Instrument nach einem der vorangehenden Ansprüche, **dadurch gekennzeichnet, dass** das Betätigungselement (14) ein zweites Kupplungsteil aufweist, welches mit einem mit dem Werkzeug (6) bewegungsgekoppelten Kupplungsteil einen Formschluss bildet.

5. Medizinisches Element nach einem der vorangehender Ansprüche, **dadurch gekennzeichnet, dass** das Betätigungselement (14) ein federvorgespanntes Rastelement (52) aufweist, das in der ersten Schaltstellung in eine erste an dem Schaft (2) ausgebildete umfängliche Ringnut (54) und in der zweiten Schaltstellung in eine zweite an dem Schaft (2) ausgebildete umfängliche Ringnut (64) eingreift.

6. Medizinisches Instrument nach einem der Ansprüche 3 bis 5, **dadurch gekennzeichnet, dass** ein mit einer Außenverzahnung versehener erster Zahnkranz (16a) das erste Kupplungsteil und ein mit einer Außenverzahnung versehener zweiter Zahnkranz (16b) das zweite Kupplungsteil des Betätigungselements (14) bildet.

7. Medizinisches Instrument nach Anspruch 5, **dadurch gekennzeichnet, dass** sich die Zähne (20) des ersten Zahnkranzes (16a) in axialer Richtung zu dem mit dem Schaft (2) bewegungsgekoppelten Kupplungsteil verjüngen und sich die Zähne (20) des zweiten Zahnkranzes (16b) in axialer Richtung zu dem mit dem Werkzeug (6) bewegungsgekoppelten Kupplungsteil verjüngen.

8. Medizinisches Instrument nach einem der Ansprüche 6 oder 7, **dadurch gekennzeichnet, dass** auf dem Schaft (2) eine erste Nabe (22, 68) mit zwei voneinander radial beabstandeten Ringabschnitten (32, 34) angeordnet ist, wobei an der Innenseite eines äußeren Ringabschnitts (34) eine mit der Verzahnung des ersten Zahnkranzes (16a) korrespondierende Verzahnung ausgebildet ist.

9. Medizinisches Instrument nach Anspruch 8, **dadurch gekennzeichnet, dass** der Schaft (2) in dem Bereich, in dem die erste Nabe (22, 68) angeordnet ist, eine Außenquerschnittskontur mit zumindest einem gerade abgeflachten Abschnitt aufweist und die von einer inneren Umfangswandung (32) begrenzte Innenquerschnittskontur (26) der ersten Nabe (22, 68) mit der Außenquerschnittskontur des Schafts (2) korrespondiert.

10. Medizinisches Instrument nach einem der Ansprüche 6 bis 9, **dadurch gekennzeichnet, dass** das Werkzeug (6) an dem distalen Ende eines in dem Schaft (2) drehbeweglich angeordneten Rohres (8) angeordnet ist und auf dem Rohr (8) eine zweite Nabe (24, 78) mit zwei voneinander radial beabstandeten Ringabschnitten (32, 34) angeordnet ist, wobei an der Innenseite eines äußeren Ringabschnitts (34) eine mit der Verzahnung des zweiten Zahnkranzes (16b) korrespondierende Verzahnung ausgebildet ist.

11. Medizinisches Instrument nach Anspruch 10, **dadurch gekennzeichnet, dass** das in dem Schaft (2) geführte Rohr (8) in dem Bereich, in dem die zweite Nabe (24, 78) an dem Rohr (8) angeordnet ist, eine Außenquerschnittskontur mit zumindest einem gerade abgeflachten Abschnitt aufweist und die von einer inneren Umfangswandung (32) begrenzte Innenquerschnittskontur der zweiten Nabe (24, 78) mit der Außenquerschnittskontur des Rohrs (8) korrespondiert.

12. Medizinisches Instrument nach einem der Ansprüche 3 bis 11, **dadurch gekennzeichnet, dass** das mit dem Schaft (2) bewegungsgekoppelte Kupplungsteil in Verschieberichtung des Betätigungselements (14) verschiebbar ist, und Formschlussmittel aufweist, mit denen es in einer Verschiebestellung mit der Handhabe (4) drehfest verbindbar ist.

13. Medizinisches Instrument nach einem der Ansprüche 3 bis 12, **dadurch gekennzeichnet, dass** das mit dem Werkzeug (6) bewegungsgekoppelte Kupplungsteil in Verschieberichtung des Betätigungselements (14) verschiebbar ist, und Formschlussmittel aufweist, mit denen es in einer Verschiebestellung mit der Handhabe (4) drehfest verbindbar ist.

14. Medizinisches Instrument nach einem der Ansprüche 3 bis 11, **dadurch gekennzeichnet, dass** das mit dem Schaft (2) bewegungsgekoppelte Kupplungsteil in Verschieberichtung des Betätigungselements (14) nicht verschiebbar ist, und Formschlussmittel aufweist, in die ein an der Handhabe (4) vorgesehenes Rastelement (74) eingreift.

15. Medizinisches Instrument nach einem der Ansprüche 3 bis 1 1 sowie 14, **dadurch gekennzeichnet, dass** das mit dem Werkzeug (6) bewegungsgekoppelte Kupplungsteil in Verschieberichtung des Betätigungselements (14) nicht verschiebbar ist, und Formschlussmittel aufweist, in die ein weiteres an der Handhabe vorgesehenes Rastelement (74) eingreift.

## Claims

1. A medical instrument with a handle (4) arranged on the proximal side, with a shank (2) which is rotatably arranged on the handle (4) and with a tool (6) which is arranged at the distal end of the shank (2) and which is rotatable relative to the shank (2), **characterised in that** an actuation element (14) is arranged on the handle (4) and is movable into at least two switch positions, wherein it is coupled in movement to the shank (2) in a first switch position, and to the tool (6) in a second switch position.

2. A medical instrument according to claim 1, **characterised in that** the actuation element (14) for the movement transmission onto the shank (2) and onto the tool (6), in each case may be rotated about a common rotation axis and is axially displaceable in the direction of the rotation axis into its switch positions.

3. A medical instrument according to one of the preceding claims, **characterised in that** the actuation element (14) comprises a first coupling element which forms a positive fit with a coupling part coupled in movement to the shank (2).

4. A medical instrument according to one of the preceding claims, **characterised in that** the actuation element (14) has a second coupling part which forms a positive fit with a coupling part coupled in movement to the tool (6).

5. A medical instrument according to one of the preceding claims, **characterised in that** the actuation element (14) comprises a spring-biased locking element (52) which in the first switch position engages into a first peripheral annular groove (54) formed on the shank (2), and in the second switch position engages into a second peripheral annular groove (64) formed on the shank (2).

6. A medical instrument according to one of the claims 3 to 5, **characterised in that** a first toothed rim (16a) provided with an outer toothing forms the first coupling part, and a second toothed rim (16b) provided with an outer toothing forms the second coupling part, of the actuation element (14).

7. A medical instrument according to claim 5, **characterised in that** the teeth (20) of the first toothed rim (16a) taper in the axial direction to the coupling part coupled in movement to the shank (2), and the teeth (20) of the second toothed rim (16b) taper in the axial direction to the coupling part coupled in movement to the tool (6).

8. A medical instrument according to one of the claims 6 or 7, **characterised in that** a first hub (22, 68) with two ring sections (32, 34) which are radially distanced to one another, is arranged on the shank (2), wherein a toothing corresponding to the toothing of the first toothed rim (16a), is formed on the inner side of an outer ring section (34).

9. A medical instrument according to claim 8, **characterised in that** the shank (2), in the region, in which the first hub (22, 68) is arranged, has an outer cross-sectional contour with at least one straight flattened section, and the inner cross-sectional contour (26) of the first hub (22, 68), said contour being delimited by an inner peripheral wall (32), corresponds to the outer cross-sectional contour of the shank (2).

10. A medical instrument according to one of the claims 6 to 9, **characterised in that** the tool (6) is arranged on the distal end of a tube (8) which is arranged in the shank (2) in a rotationally moveable manner, and a second hub (24, 78) with two ring sections (32, 34) which are radially distanced to one another, is arranged on the tube (8), wherein a toothing corresponding to the toothing of the second toothed rim (16b), is formed on the inner side of an outer ring section (34).

11. A medical instrument according to claim 10, **characterised in that** the tube (8) guided in the shank (2), in the region in which the second hub (24, 78) is arranged on the tube (8), has an outer cross-sectional contour with at least one straight, flattened section, and the inner cross-sectional contour of the second hub (24, 78), said contour being delimited by an inner peripheral wall (32), corresponds to the outer cross-sectional contour of the tube (8).

12. A medical instrument according to one of the claims 3 to 11, **characterised in that** the coupling part coupled in movement to the shank (2), is displaceable in the displacement direction of the actuation element (14), and comprises positive fit means, with which, in one displacement position, it may be connected in a rotational fixed manner to the handle (4).

13. A medical instrument according to one of the claims 3 to 12, **characterised in that** the coupling part coupled in movement to the tool (6) is displaceable in the displacement direction of the actuation element (14), and comprises positive fit means with which, in one displacement position, it may be connected in a rotationally fixed manner to the handle (4).

14. A medical instrument according to one of the claims 3 to 11, **characterised in that** the coupling part which is coupled in movement to the shank (2), is not displaceable in the displacement direction of the actuation element (14), and comprises positive fit means, into which a locking element (74) provided on the handle (4) engages.

15. A medical instrument according to one of the claims 3 to 11, as well as 14, **characterised in that** the coupling part which is coupled in movement to the tool (6), is not displaceable in the displacement direction of the actuation element (14), and comprises positive fit means, into which a further locking element (74) provided on the handle engages.

## Revendications

1. Instrument médical comprenant une manette (4) disposée côté proximal, comportant une tige (2) pouvant pivoter au niveau de la manette (4) et présentant un outil (6) disposé au niveau de extrémité distale de la tige (2), lequel peut pivoter par rapport à la tige (2), **caractérisé en ce qu'**au niveau de la manette (4) est disposé un élément d'actionnement (14) qui est mobile dans au moins deux positions de commutation, suite à quoi il est couplé par déplacement avec la tige (2) dans une première position de commutation et avec l'outil (6) dans une seconde position de commutation.

2. Instrument médical selon la revendication 1, **caractérisé en ce que** l'élément d'actionnement (14) peut pivoter respectivement autour d'un axe de rotation commun pour une transmission de mouvement sur la tige (2) et sur l'outil (6), et peut être déplacé axialement dans la direction de l'axe de rotation dans ses positions de commutation.

3. Instrument médical selon l'une des revendications précédentes, **caractérisé en ce que** l'élément d'actionnement (14) présente une première pièce d'accouplement qui forme une liaison avec correspondance de forme avec une pièce d'accouplement couplée par déplacement avec la tige (2).

4. Instrument médical selon l'une des revendications précédentes, **caractérisé en ce que** l'élément d'actionnement (14) présente une seconde pièce d'accouplement qui forme une liaison avec correspondance de forme avec une pièce d'accouplement couplée par déplacement avec l'outil (6).

5. Instrument médical selon l'une des revendications précédentes, **caractérisé en ce que** l'élément d'actionnement (14) présente un élément d'enclenchement précontraint par ressort (52), qui dans la première position de commutation s'engrène dans une première rainure annulaire (54) périphérique exécutée au niveau de la tige (2), et dans une seconde position de commutation s'engrène dans une seconde rainure annulaire périphérique (64) exécutée au niveau de la tige (2).

6. Instrument médical selon l'une des revendications 3 à 5, **caractérisé en ce qu'**une première couronne dentée (16a) munie d'une denture externe forme la première pièce d'accouplement et une seconde couronne dentée (16b) munie d'une denture externe forme la seconde pièce d'accouplement de l'élément d'actionnement (14).

7. Instrument médical selon la revendication 5, **caractérisé en ce que** les dents (20) de la première couronne dentée (16a) se rétrécissent dans la direction axiale vers la pièce d'accouplement couplée par déplacement avec la tige (2) et **en ce que** les dents (20) de la seconde couronne dentée (16b) se rétrécissent dans la direction axiale vers la pièce d'accouplement couplée par déplacement avec l'outil (6).

8. Instrument médical selon l'une des revendications 6 ou 7, **caractérisé en ce que** sur la tige (2) est disposé un premier moyeu (22, 68) comportant deux segments annulaires (32, 34) écartés radialement l'un de l'autre, une denture correspondant à la denture de la première couronne dentée (16a) étant exécutée au niveau de la face interne du segment annulaire externe (34).

9. Instrument médical selon la revendication 8, **caractérisé en ce que** la tige (2), dans la zone dans laquelle est disposé le premier moyeu (22, 68), présente un contour externe en coupe transversale ayant au moins un segment droit aplati, et **en ce que** le contour interne en coupe transversale (26), délimité par une paroi périphérique interne (32) du premier moyeu (22, 68), correspond au contour externe en coupe transversale de la tige (2).

10. Instrument médical selon l'une des revendications 6 à 9, **caractérisé en ce que** l'outil (6) est disposé au niveau de l'extrémité distale d'un tube (8) disposé dans la tige (2) de façon à pouvoir tourner, et **en ce que** sur le tube (8) est disposé un second moyeu (24, 78) comportant deux segments annulaires (32, 34) écartés radialement l'un de l'autre, une denture correspondant à la denture de la seconde couronne dentée (16b) étant exécutée au niveau de la face interne d'un segment annulaire externe (34) .

11. Instrument médical selon la revendication 10, **caractérisé en ce que** le tube (8) guidé dans la tige (2) présente, dans la zone dans laquelle le second moyeu (24, 78) est disposé au niveau du tube (8), un contour externe en coupe transversale présentant au moins un segment droit aplati, et **en ce que** le contour interne en coupe transversale, délimité par une paroi périphérique interne (32), du second moyeu (24, 78), correspond au contour externe en coupe transversale du tube (8).

12. Instrument médical selon l'une des revendications 3 à 11, **caractérisé en ce que** la pièce d'accouplement couplée par déplacement avec la tige (2) est mobile dans la direction de déplacement de l'élément d'actionnement (14), et présente des moyens de liaison avec correspondance de forme à l'aide desquels elle peut, dans une position de déplacement, être reliée avec la manette (4) sans pouvoir pivoter.

13. Instrument médical selon l'une des revendications 3 à 12, **caractérisé en ce que** la pièce d'accouplement couplée en déplacement à l'outil (6) est mobile dans la direction de déplacement de l'élément d'actionnement (14), et présente des moyens de liaison avec correspondance de forme à l'aide desquels elle peut, dans une position de déplacement, être reliée à la manette (4) sans pouvoir pivoter.

14. Instrument médical selon l'une des revendications 3 à 11, **caractérisé en ce que** la pièce d'accouplement couplée en déplacement à la tige (2) n'est pas mobile dans la direction de déplacement de l'élément d'actionnement (14) et présente des moyens de liaison avec correspondance de forme dans lesquels s'engrène un élément d'enclenchement (74) prévu dans la manette (4).

15. Instrument médical selon l'une des revendications 3 à 11 ainsi que 14, **caractérisé en ce que** la pièce d'accouplement couplée par déplacement avec l'outil (6) n'est pas mobile dans la direction de déplacement de l'élément d'actionnement (14) et présente des moyens de liaison avec correspondance de forme dans lesquels s'engrène un autre élément d'enclenchement (74) prévu dans la manette.
